# EUROPEAN PATENT APPLICATION

(11) **EP 0 988 869 A2**
(43) Date of publication of application: **29.03.2000**
(21) Application number: 99116228.0
(22) Date of filing: 17.08.1999
(51) Int. Cl.: A61M 16/00

(54) **Breathing apparatus**

(30) Priority: 25.09.1998 SE 9803261
(71) Applicant: Siemens-Elema AB, 171 95 Solna 1 (SE)
(72) Inventor: Cardell, Mats, 170 63 Solna (SE)

(57) **Abstract**

A breathing apparatus (4), devised for connection to a patient (2), comprising an alarm system (8) for generating an alarm if a fault is detected in the operation of the breathing apparatus (4), is described. To prevent the needless triggering of an alarm whenever the patient (2) speaks, the breathing apparatus (4) according to the invention is equipped with a manual control unit (12) devised, when operated, primarily by the patient (2), to deactivate the alarm system's (8) generation of an alarm.

## Description

The present invention relates to a breathing apparatus according to the preamble to claim 1.

Since breathing apparatuses, such as ventilators and respirators, are frequently used for life support purposes, their safety is the subject of stringent demands. Most breathing apparatuses for medical usage incorporate an alarm system. The alarm system is devised to activate an alarm whenever a fault is detected in the device's operation. Alarms are sounded for a plurality of different faults. Some have high priority, since there are faults with a direct impact on the device's life support functions. So other faults have lower priority.

Many patients are connected to the breathing apparatus by a tracheal tube. Some tracheal tubes are inserted into the trachea past the vocal cords others are not. Both ways make it somewhat difficult for patients to speak, i.e. those patients who want to and are strong enough to speak. However, the patient is able to speak, but gas must then be allowed leak out alongside the tracheal tube in order to properly pass the vocal cords. A problem then arises. Most respirators compare inhaled and exhaled volumes in order to determine leaks in the system. The alarm system therefore interprets the leakage of gas alongside the tracheal tube as a fault, and an alarm is sounded. This alarm is loud and intense and renders virtually every kind of verbal communication more difficult. Being forced to turn off the breathing apparatus's needless alarms and reset the alarm system is also very annoying for the staff.

Another situation of unnecessary alarms normally occurs when the patient coughs. A pressure alarm may then sound, requiring personnel to switch the alarm off.

One objective of the present invention is to achieve a breathing apparatus that solves the aforementioned problems.

This objective is achieved according to the invention when the breathing apparatus is devised as is evident from the characterising part of claim 1.

Advantageous embodiments are evident from the dependent claims of claim 1.

Using a manual control unit, the patient is able to momentarily inhibit the alarm system's generation of an alarm in order to speak. The alarm system does therefore not generate a sound. An undisturbed conversation can thus be held.

In a similar manner can the conscious patient inhibit a pressure alarm when he/she needs to cough.

Since the actions that might have caused an alarm is consciously made, they are also known to be imminent.

However, registration of inhibitions and the reason for inhibition (leakage, pressure, etc.) can very well take place (and even be visually indicated as alarms). This provides the personnel with tools for further viewing the status of the patient as well as the patient-machine system.

To make things easier for the patient, the manual control unit can be connected to the breathing apparatus by a cord, or it can have a wireless connection to the breathing apparatus.

The alarm system is appropriately kept deactivated as long as the patient operates the manual unit, but limiting deactivation to a specific period of time may be appropriate. In case of speaking, there will most likely be effects on expiration times as well as indication of leakage. In order to ensure that the inhibition of the alarm is not faulty, requirements of active action from the patient each expiration phase can be used as a safety measure.

Moreover, deactivation should be limited to certain types of faults, in particular faults that the patient can cause by his/her conscious actions.

The manual control unit can comprise a push-button, lever or some other device for starting activation of the function. It could even be controlled by eye movements or similar for more or less completely paralysed patients.

One embodiment of a breathing apparatus according to the invention will be described below in greater detail, referring to the figure.

The figure shows a patient 2 connected to a breathing apparatus 4. The breathing apparatus can consist of a ventilator, anaesthesia machine or some other medical respiratory device. The patient 2 is connected to the breathing apparatus 2 by a tubing system 6 including a tracheal tube (not shown).

An alarm system 8 is arranged in the breathing apparatus 4. The alarm system 8 generates an alarm whenever a fault is detected in the operation of the breathing apparatus 4. The alarm is emitted through a loudspeaker 10.

Leakage of gas from the tubing system 6 is one type of alarm for which the alarm system 8 sounds an alarm. If the patient 2 speak, gas leaks out past the tracheal tube, and the alarm system 8 will respond by generating an unnecessary and annoying alarm.

Another unnecessary and annoying alarm is a high pressure alarm caused by coughing.

To keep the unnecessary alarms from sounding, the breathing device 4 according to the invention is equipped with a manual control unit 12. The manual control unit 12 is connected to the breathing apparatus 4 and the alarm system 8 by a cord 14. The cord 14 can be replaced a wireless transmission capability, e.g. using IR light, ultrasound or some other wireless means.

Whenever the patient 2 wishes to speak, she/he operates the manual control unit 12, e.g. by pressing a button, thereby deactivating the alarm system's generation of a leakage alarm. Similarly, high pressure alarms caused by coughing can be avoided by patient 2 if she/he presses the (same or another) button before coughing.

It should be noted that the manual control unit 12 could use any known means for operating it. Instead of a button it could contain other kinds of switches. It could also (particularly for paralysed patients) contain a blow-off nozzle, neck controlled switch, etc.

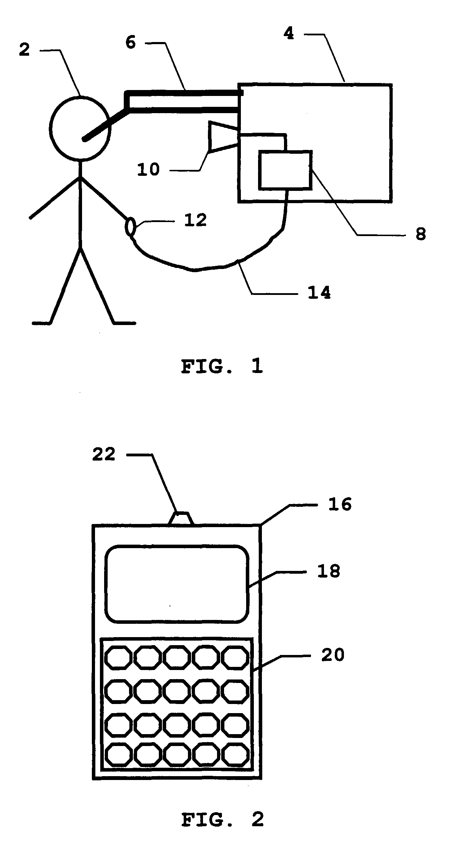

As shown in FIG. 2, the manual control unit 16 could also comprise a small screen 18 and keyboard 20. By punching different codes on the keyboard 20, different alarm types can be selected (shown in the screen) for inhibition. In FIG. 2 the manual control unit 16 is shown to have an IR-lamp 22 for wireless communication with the alarm system.

Deactivation can last as long as the patient 2 operates the manual control unit 12 or for a specific period of time.

For many instances it could on the other hand be more advantageous in relation to patient safety if the manual control unit 12, 16 had to be activated for every occurrence of an action.

For instance, normally a person exhales when she/he speaks. The deactivation could therefore be configured to deactivate the leakage alarm only for one expiration phase at a time. The alarm would thus be activated again at the following inspiration phase. Similarly, coughing causes a short time period of increased pressure. Deactivation could be devised to last only one such period at a time.

It can also be specified that deactivation is only necessary for the audible alarm. In other words, visual indications can show that the alarm system 8 has recognised situations that normally would give rise to alarms have occurred. Attending personnel can then, at their own convenience, check which type of alarm situations that has been identified by the alarm system and hove many that has been inhibited by the patient.

It is of course also possible for the personnel to activate the manual control unit 12, 16 when necessary. The manual control unit 12, 16 could also be devised to allow one group of alarms to be inhibited by the patient and another group of alarms to be inhibited by personnel.

## Claims

1. A breathing apparatus (4), devised for connection to a patient (2), comprising an alarm system (8) for identifying faults in the operation of the breathing apparatus (4) and generating an alarm if a fault is detected), **characterised by** a manual control unit (12; 16), devised, when operated, preferably by the patient (2), to inhibit the alarm system's (8) generation of an alarm of an imminent fault.

2. The breathing apparatus according to claim 1, **characterised in** that the manual control unit (12) is connected to the breathing apparatus (4) by a cord (14).

3. The breathing apparatus according to claim 1, **characterised in** that the manual control unit (16) is devised with means (22) for wireless communication with the breathing apparatus (4), preferably by means of infrared light.

4. The breathing apparatus according to any of the above claims, **characterised in** that the manual control unit (12) is devised so the alarm system's (8) generation of an alarm remains inhibited as long as the manual control unit (12) is operated.

5. The breathing apparatus according to any of the above claims, **characterised in** that the manual control unit's (12) inhibition of the alarm system's (8) generation of an alarm is limited to a specific event.

6. The breathing apparatus according to any of the above claims, **characterised in** that the manual control unit's (12) inhibition of the alarm system's (8) generation of an alarm is limited to a predetermined period of time.

7. The breathing apparatus according to any of the above claims, **characterised in** that the manual control unit's (12) inhibition of the alarm system's (8) generation of an alarm is limited to one or more predetermined types of alarm, a leakage alarm or pressure alarm in particular.

8. The breathing apparatus according to any of the above claims, **characterised in** that the manual control unit (12) is devised with a switch.

9. The breathing apparatus according to any of the claims 1-7, **characterised in** that the manual control unit (16) is devised with a key-board (20) and screen (18).
